(19) Europäisches Patentamt
European Patent Office
Office européen des brevets

(11) **EP 4 732 945 A1**

(12) **EUROPEAN PATENT APPLICATION**
published in accordance with Art. 153(4) EPC

(43) Date of publication:
29.04.2026 Bulletin 2026/18

(21) Application number: 24865921.1

(22) Date of filing: 13.09.2024

(51) International Patent Classification (IPC):
*B01J 35/30* (2024.01)    *B01J 35/40* (2024.01)
*B01J 23/745* (2006.01)    *C07C 1/12* (2006.01)
*C10G 2/00* (2006.01)

(52) Cooperative Patent Classification (CPC):
B01J 23/745; B01J 23/881; B01J 35/30;
B01J 35/40; B01J 35/45; B01J 35/53; C07C 1/12;
C10G 2/00

(86) International application number:
PCT/KR2024/014075

(87) International publication number:
WO 2025/058482 (20.03.2025 Gazette 2025/12)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 15.09.2023 KR 20230123492

(71) Applicant: LG Chem, Ltd.
Seoul 07336 (KR)

(72) Inventors:
• PARK, Seung Won
  Daejeon 34122 (KR)
• KIM, Won Hee
  Daejeon 34122 (KR)

• JUNG, Da Won
  Daejeon 34122 (KR)
• KIM, Seong Min
  Daejeon 34122 (KR)
• KIM, Eui Tae
  Daejeon 34122 (KR)
• MOON, Ji Won
  Daejeon 34122 (KR)
• LEE, Yong Hee
  Daejeon 34122 (KR)
• KIM, Ki Hwan
  Daejeon 34122 (KR)

(74) Representative: Hoffmann Eitle
Patent- und Rechtsanwälte PartmbB
Arabellastraße 30
81925 München (DE)

(54) **PARTICLE**

(57) The present specification discloses particles. The particles can be applied to a reaction for forming hydrocarbons from a raw material including $CO_2$ and/or CO. The present specification is intended to disclose the particles, wherein the particles can obtain desired products with excellent selectivity and conversion ratios without causing undesirable agglomeration or sintering phenomena and deterioration of catalytic activity during reaction processes. For example, the particles can obtain hydrocarbons having 5 or more carbon atoms with high selectivity and conversion ratios in a reaction for obtaining hydrocarbons from a raw material including $CO_2$ and/or CO. The present specification also discloses a use of the particles.

EP 4 732 945 A1

[Figure 5]

## Description

### Technical Field

This application claims the benefit of priority based on Korean Patent Application No. 10-2023-0123492 dated September 15, 2023, the disclosure of which is incorporated herein by reference in its entirety.

The present specification discloses particles. In one example, the particles may be a catalyst applied to a reaction of forming hydrocarbons using $CO_2$ as a raw material.

### Background Art

$CO_2$ emitted in energy production processes based on fossil fuels is considered as a major cause of climate changes such as global warming. Various attempts for capturing and utilizing $CO_2$ exist, and the catalytic $CO_2$ hydrogenation reaction disclosed in Non-Patent Document 1 is one of such attempts. These reactions utilize methods such as FTS (Fischer-Tropsch) or MTO (Methanol-to-Olefin), and the methods reported to date can be largely classified into two types.

A first type is a method of producing hydrocarbons such as liquid fuel, olefins, and aromatic compounds through the FTS (Fischer-Tropsch) reaction after the RWGS (Reverse Water-Gas Shift) reaction converting $CO_2$ to CO using a Fe catalyst (Non-Patent Document 2). A second type is a method of converting $CO_2$ to methanol using a catalyst ($In_2O_3$ or Cu/ZnO/$Al_2O_3$ series) and producing high value-added products (e.g. gasoline, etc.) through additional reactions (Non-Patent Document 3).

In the first type, the RWGS reaction is an endothermic reaction, and the FTS reaction is an exothermic reaction. Therefore, in order that the RWGS reaction proceeds efficiently, a lot of energy must be supplied, and to prevent the deterioration of the catalyst activity in the FTS reaction, the reaction must proceed while effectively removing the reaction heat.

However, it is a difficult problem to continuously perform such energy supply and energy removal. Particularly, because the removal efficiency of energy generated in the FTS reaction is lowered, problems caused by undesirable agglomeration phenomena of catalyst particles, and lowering and/or deterioration of the catalyst activity frequently occur, and these problems lead to low selectivity and conversion rate for hydrocarbons.

For example, in order that the products are applied as so-called aviation fuels, and the like, hydrocarbons having a certain number of carbon atoms must be present in the product, but if the selectivity and conversion rate are lowered, the product does not contain the desired hydrocarbon or the ratio of the hydrocarbon decreases.

<Prior Art Documents>

<Non-Patent Documents>

(Non-Patent Document 1) Ra, E. C.; Kim, K. Y.; Kim, E. H.; Lee, H.; An, K.; Lee, J. S. Recycling Carbon Dioxide through Catalytic Hydrogenation: Recent Key Developments and Perspectives. ACS Catal. 2020, 10 (19), 11318-11345. DOI: 10.1021/acscatal.0c02930

(Non-Patent Document 2) Choi, Y. H.; Jang, Y. J.; Park, H.; Kim, W. Y.; Lee, Y. H.; Choi, S. H.; Lee, J. S. Carbon Dioxide Fischer-Tropsch Synthesis: A New Path to Carbon-Neutral Fuels. Appl. Catal., B 2017, 202, 605-610, DOI: 10.1016/j.apcatb.2016.09.072

(Non-patent Document 3) Sharma, P.; Sebastian, J.; Ghosh, S.; Creaser, D.; Olsson, L. Recent Advances in Hydrogenation of CO2 into Hydrocarbons via Methanol Intermediate over Heterogeneous Catalysts. Catal. Sci. Technol. 2021, 11 (5), 1665-1697, DOI: 10.1039/D0CY01913E

## Disclosure

### Technical Problem

The present specification discloses particles. The particles can be applied to a reaction for forming hydrocarbons from a raw material including $CO_2$ and/or CO. The present specification is intended to disclose the particles, wherein the particles can obtain desired products with excellent selectivity and conversion ratios without causing undesirable agglomeration or sintering phenomena and deterioration of catalytic activity during reaction processes. For example, the particles can obtain hydrocarbons having 5 or more carbon atoms with high selectivity and conversion ratios in a reaction for obtaining hydrocarbons from a raw material including $CO_2$ and/or CO. The present specification also discloses a use of the particles.

**Technical Solution**

Among physical properties described in this specification, the physical property affected by a measurement temperature is a physical property measured at room temperature, unless otherwise specified.

The term room temperature means a natural temperature without artificially heating or cooling, which may mean, for example, any one temperature within a range of 10°C to 30°C, or a temperature of about 23°C or about 25°C or so.

In this specification, the unit of temperature is Celsius (°C), unless otherwise specified.

Among physical properties mentioned in this specification, the physical property affected by a measurement pressure is a physical property measured at normal pressure, unless otherwise specified.

The term normal pressure means a natural pressure without artificially pressurizing or depressurizing, which may usually mean any one pressure within a range of about 730 mmHg to 790 mmHg.

Among physical properties mentioned in this specification, the physical property affected by measurement humidity is a physical property measured at standard state humidity, unless otherwise specified. The standard state humidity is any one relative humidity (RH%, Relative Humidity) within a range of 40% to 60%, which means, for example, a relative humidity (RH%, Relative Humidity) of about 55%, or 60% or so.

The term center coordinate or center mentioned in this specification means the center of mass, unless otherwise specified.

The present specification discloses particles.

The particle may be a catalyst particle, and in one example, may be a $CO_2$ or CO conversion catalyst or hydrogenation catalyst particle. Such a catalyst may be applied to a reaction for producing hydrocarbon compounds using a raw material including $CO_2$ and/or CO. At this time, the reaction may be a so-called RWGS (Reverse Water-Gas Shift) reaction and/or FTS (Fischer-Tropsch) reaction.

The particles may be in the form of secondary particles formed by agglomeration of a plurality of primary particles. The desired effect may be obtained by adjusting the form of the secondary particles.

For example, in the particles in the form of the secondary particles as above, the primary particles may be particles of any metal (hereinafter, first metal) or particles including the first metal. In addition, at least some primary particles among the plurality of primary particles forming the secondary particles may comprise a metal (hereinafter, second metal) different from the first metal on the surface. That is, the second metal may exist on the surface of the at least some primary particles, and the second metal may form a continuous or discontinuous layer on the surface of the primary particles, or may be included in the layer.

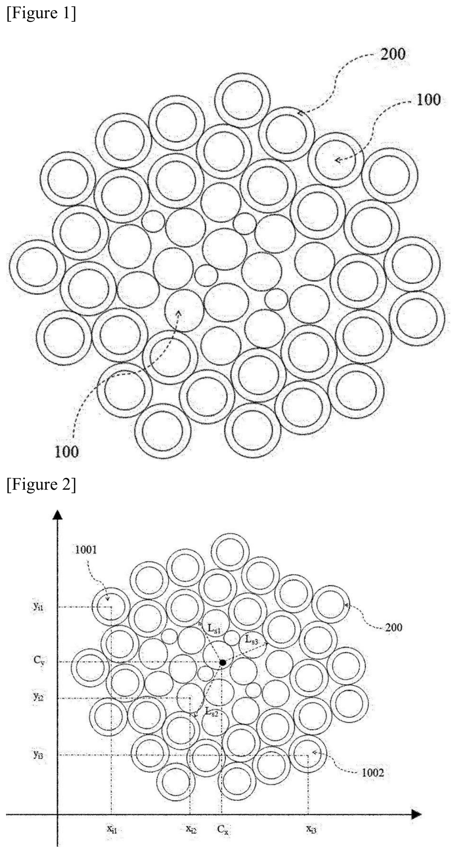

Figure 1 is an exemplary representation of a cross-section of the particle.

As in the drawing, the particle may be in the form of a secondary particle formed by agglomeration of a plurality of primary particles (100), and the second metal may exist on the surface of at least some primary particles (100) among the plurality of primary particles (100).

In the drawing, the shape of the primary particle (100) is shown as being approximately circular, but the actual shape of the primary particle may also be a shape other than circular, such as an irregular shape. Also, although the drawing shows a case where the second metal forms a continuous layer (200) on the surface of the primary particle (100), the second metal may form a discontinuous layer on the surface of the primary particle (100), or may exist only on a part of the surface of the primary particle (100). In addition, although the drawing shows a form where only the primary particles (100) are aggregated, other elements (for example, supports or molds, etc.) may also exist within the secondary particle, and empty spaces where the primary particles or other elements do not exist may also exist within the secondary particle.

As exemplarily shown in Figure 1, the second metal may exist on at least the surfaces of the primary particles distributed on the surface of the secondary particle among the plurality of primary particles forming the secondary particle. In addition, on the drawing, the layers (200) by the second metal are present only on the surfaces of the primary particles present toward the surface of the secondary particle, but in some cases, the layers by the second metal may also be formed on the surfaces of most primary particles.

By controlling the shape of the secondary particle, and the like in the following manner, the secondary particle may exhibit desired catalytic activity, and furthermore, such catalytic activity may be stably maintained during the reaction process.

For example, in a cross-section of the secondary particle, the ratio B/A of the area B of the second metal region to the area A of the first metal region may be within a predetermined range.

Here, the area A of the first metal region and the area B of the second metal region mean regions identified by the first metal and the second metal, for example, in a cross-sectional image of the secondary particle, such as a SEM (Scanning Electron Microscope) image or a TEM (Transmission Electron Microscope) image. Such regions may be displayed based on a DBSCAN (Density-based spatial clustering of applications with noise) algorithm for the cross-sectional image. The specific details of designating such regions are summarized in "Test Example 1" of this specification.

Hereinafter, in this specification, for the convenience of explanation, it is assumed that the circular shape in Figures 1 and 2, etc. is the region of the first metal, and it is assumed that the region existing outside some circular shapes among the circular shapes is the region of the second metal.

The lower limit of the ratio B/A may be 0, 0.01, 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, or 1 or so, and the upper limit thereof may be 10, 8, 6, 4, 2, 1.7, 1.6, 1.5, 1.4, 1.3, 1.2, 1.1, 1.0, 0.9, 0.8, 0.7, 0.6, 0.5, 0.4, 0.3, 0.2, or 0.15 or so. The ratio B/A may be within a range of less than or equal to, or less than any one upper limit arbitrarily selected from the above-listed upper limits while being more than or equal to, or more than any one lower limit arbitrarily selected from the above-listed lower limits. By designing the catalyst to satisfy this range, the desired catalytic activity may be obtained, and such catalytic activity may be stably maintained during the reaction process.

The lower limit of the area A in the ratio B/A may be 200, 400, 600, 800, 850, 900, 1,000, 5,000, 10,000, 50,000, 100,000, or 150,000 or so, and the upper limit thereof may be 1,000,000, 500,000, 100,000, 50,000, 10,000, 5,000, 1,000, or 950 or so. The area A may be within a range of less than or equal to, or less than any one upper limit arbitrarily selected from the above-listed upper limits while being more than or equal to, or more than any one lower limit arbitrarily selected from the above-listed lower limits. By designing the catalyst to satisfy this range, the desired catalytic activity may be obtained, and such catalytic activity may be stably maintained during the reaction process. The unit of the area A is $\mu m^2$.

The catalyst may be designed so that $R_v$ of Equation 3 below is within a predetermined range.

$$[\text{Equation 3}]$$

$$R_v = \frac{-L_s + \sqrt{L_s^2 + \dfrac{B}{\pi}}}{2 \times \sqrt{\dfrac{A}{\pi}}}$$

In Equation 3, $L_s$ may be an average distance from the center of the first metal region of the cross-section of the secondary particle to the second metal region. The unit of this $L_s$ may be $\mu m$ upon application of Equation 3.

In Equation 3, A is A at the ratio B/A, and B is B at the ratio B/A. Upon application of Equation 3, the units of the ratios A and B are each $\mu m^2$.

The $L_s$ may be confirmed in the following manner.

First, the center of the cross-section of the secondary particle is designated.

The center of the cross-section of the secondary particle may be defined by $C_x$ of Equation 1 below and $C_y$ of Equation 2 below.

$$[\text{Equation 1}]$$

$$C_x = \frac{\sum_{i=1}^{n}(x_i \times A_i)}{\sum_{i=1}^{n} A_i}$$

$$[\text{Equation 2}]$$

$$C_y = \frac{\sum_{i=1}^{n}(y_i \times A_i)}{\sum_{i=1}^{n} A_i}$$

The $C_x$ and $C_y$ are coordinates designated in the cross-section of the secondary particle.

To designate the $C_x$ and $C_y$, the cross-section of the secondary particle is first positioned on the xy coordinates, as exemplarily shown in Figure 2. Thereafter, the first metal region (the part of the cross-section of the secondary particle occupied by the first metal) and the second metal region (the part of the cross-section of the secondary particle occupied by the second metal) are distinguished and indicated within the individual multiple primary particles forming the secondary particle. Through this operation, the region of the first metal appears in the form of multiple primary particles on the drawing, and the region of the second metal is confirmed at the outside of some regions among the regions of the first metal. The method of designating these regions is as described above and is specifically described in "Test Example 1" of this specification.

Subsequently, the center coordinates of the individual regions of the first metal are recorded in the x-coordinate and y-coordinate. For example, in Figure 2, the center coordinates of the region 1001 of the first metal are indicated as $(x_{i1}, y_{i1})$, and the center coordinates of the region 1002 of the second metal are indicated as $(x_{i3}, y_{i3})$. Here, the center coordinates are the coordinates where the center of mass of the relevant region of the first metal exists.

For all regions of the first metal confirmed in the drawing, the center coordinates are indicated in the above manner, and the area of the region of the first metal having the respective center coordinates is also obtained.

This operation is performed for all regions of the first metal in the form of primary particles.

Thereafter, as indicated in Equation 1 above, values are obtained for all regions of the first metal, wherein a value is obtained by multiplying the value of the x-coordinate of the region of the first metal in the form of the primary particle by the area of the region of the first metal, and the obtained values are added up, and then the value $(C_x)$ obtained by dividing the value by the sum of the areas of the individual regions of the first metal becomes the x-coordinate of the center of the cross-section of the secondary particle.

As indicated in Equation 2, values are obtained for all regions of the first metal, wherein a value is obtained by multiplying the value of the y-coordinate of the region of the first metal in the form of the primary particle by the area of the region of the first metal, and the obtained values are added up, and then the value $(C_y)$ obtained by dividing the value by the sum of the areas of the individual regions of the first metal becomes the y-coordinate of the center of the cross-section of the secondary particle.

The coordinates $(C_x, C_y)$ of this center are indicated in Figure 2.

Therefore, in Equations 1 and 2 above, $x_i$ is the x-coordinate of the center of the region of the first metal in the primary particle cross-sections, respectively, in the cross-section of the secondary particle, and $y_i$ is the y-coordinate of the center of the region of the first metal in the primary particle cross-sections, respectively, in the cross-section of the secondary particle.

In addition, in Equations 1 and 2, $A_i$ is the area of the region of the first metal in the primary particle cross-section having the coordinates $x_i$ and $y_i$.

Such center coordinates $x_i$ and $y_i$, and the area $A_i$ may be obtained, for example, using a Python program, and may be obtained, for example, using a library such as scipy or numpy in the Python program. In such a program, the region of the first metal is divided into a plurality of small polygons such as triangles or quadrangles, and the center coordinates $x_i$ and $y_i$ and the area $A_i$ are obtained through a computation process corresponding to Equations 1 and 2 above using the areas and center coordinates of the regions of the respective divided polygons.

In addition, in Equations 1 and 2, n is the number of regions of the first metal, which corresponds to the number of primary particles forming the secondary particles. When the first and second metal regions are divided into the plurality of polygons, n may be the number of the divided polygons. Usually, according to an algorithm such as a Python program, an appropriate number of polygons capable of producing the optimal result are formed, and thus, the range of n is determined according to the Python program, which is not particularly limited.

After obtaining the center coordinates $(C_x, C_y)$ of the secondary particle cross-section, the shortest distance from the center coordinates to the region where the second metal exists is obtained. In Figure 2, the shortest distances are indicated as $L_{s1}$, $L_{s2}$, and $L_{s3}$. In Figure 2, only three distances $(L_{s1}, L_{s2}, L_{s3})$ are indicated, but the shortest distances are indeed obtained while scanning in all directions 360 degrees from the center coordinates $(C_x, C_y)$. Thereafter, the obtained shortest distances are arithmetically averaged, whereby the result may be used as the $L_s$. At this time, the unit of $L_s$ may be $\mu$m. Such an average distance $L_s$ may also be obtained using the Python program, and for example, it may be obtained using a library such as scipy or numpy in the Python program.

The lower limit of $R_v$ of Equation 3 above obtained through the values obtained in this way may be 0.01, 0.03, 0.05, 0.07, 0.09, 0.1, 0.2, 0.3, 0.4, or 0.5 or so, and the upper limit thereof may be 1.5, 1.3, 1.1, 1.0, 0.5, 0.1, 0.08, 0.06, 0.05, or 0.04 or so. The $R_v$ may be within a range of less than or equal to, or less than any one upper limit arbitrarily selected from the above-listed upper limits while being more than or equal to, or more than any one lower limit arbitrarily selected from the above-listed lower limits. By designing the catalyst to satisfy this range, the desired catalytic activity may be obtained, and such catalytic activity may be stably maintained during the reaction process.

Here, the lower limit of $L_s$ may be 0, 30, 45, 50, 70, 90, 100, 150, 200, 300, or 400 or so, and the upper limit thereof may be 1,000, 900, 800, 700, 600, 500, 450, 400, 300, 250, 200, 150, 100, 80, or 70 or so. The $L_s$ may be within a range of less than or equal to, or less than any one upper limit arbitrarily selected from the above-listed upper limits while being more than or equal to, or more than any one lower limit arbitrarily selected from the above-listed lower limits. By designing the catalyst to satisfy this range, the desired catalytic activity may be obtained, and such catalytic activity may be stably maintained during the reaction process. The unit of the $L_s$ is $\mu m$.

The catalyst may be designed so that $R_g$ of Equation 4 below is within a predetermined range.

[Equation 4]

$$R_g = \frac{-L_s + \sqrt{L_s^2 + \dfrac{B}{\pi}}}{r + a}$$

In Equation 4, $L_s$ is the same as $L_s$ in Equation 3 above, and its unit is $\mu m$. In addition, B in Equation 4 is B in the ratio B/A. Upon application of Equation 4, the unit of the area B is each $\mu m^2$.

In Equation 4, r is a value obtained by Equation 5 below.

[Equation 5]

$$\frac{r}{2} = \sqrt{\frac{A_{avg}}{\pi}}$$

In Equation 5, $A_{avg}$ is an arithmetic mean of the areas of the first metal regions belonging to the respective primary particles in the cross-section of the secondary particle. The method of obtaining this arithmetic mean is described in Test Example 1 of this specification, and when obtaining r in Equation 5, the value of $A_{avg}$ in the unit of $\mu m^2$ is applied.

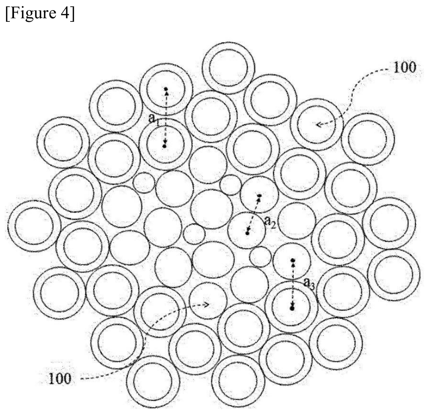

In Equation 4, a is an average distance between the centers of the first metal regions in the cross-sections of the primary particles. In Figure 4, the spacings between the centers of the first metal regions (100) are indicated as $a_1$, $a_2$, and $a_3$. The spacings between the centers may be obtained for all the first metal regions (100), and the arithmetic mean of the obtained values may be used as a in Equation 4. Upon application of Equation 4, the value of a in the unit of $\mu m$ is applied. This a may be obtained using a k-Nearest Neighbors algorithm.

The lower limit of $R_g$ in Equation 4 may be 100, 200, 250, 300, 350, 400, 500, 550, 600, 650, 700, 750, 800, 850, 900, or 950 or so, and the upper limit thereof may be 2,000, 1,500, 1,000, 800, 600, 400, or 300 or so. The $R_g$ may be within a range of less than or equal to, or less than any one upper limit arbitrarily selected from the above-listed upper limits while being more than or equal to, or more than any one lower limit arbitrarily selected from the above-listed lower limits. By designing the catalyst to satisfy this range, the desired catalytic activity may be obtained, and such catalytic activity may be stably maintained during the reaction process.

The range of the ratio a/r of a to r in Equation 4 may be adjusted.

The lower limit of the ratio a/r may be 0.1, 0.3, 0.5, 0.7, 0.9, 1.1, or 1.3 or so, and the upper limit thereof may be 2, 1.8, 1.6, 1.5, 1.4, or 1.3 or so. The a/r may be within a range of less than or equal to, or less than any one upper limit arbitrarily selected from the above-listed upper limits while being more than or equal to, or more than any one lower limit arbitrarily selected from the above-listed lower limits. By designing the catalyst to satisfy this range, the desired catalytic activity may be obtained, and such catalytic activity may be stably maintained during the reaction process.

The lower limit of r in Equation 4 may be 1, 3, 5, 7, 9, 10, 15, 20, 25, or 27 or so, and the upper limit thereof may be 500, 450, 400, 350, 300, 250, 200, 150, 100, 80, 60, 40, 35, 30, 25, or 20 or so. The r may be within a range of less than or equal to, or less than any one upper limit arbitrarily selected from the above-listed upper limits while being more than or equal to, or more than any one lower limit arbitrarily selected from the above-listed lower limits. By designing the catalyst to satisfy this range, the desired catalytic activity may be obtained, and such catalytic activity may be stably maintained during the reaction process. The unit of the r is nm. This r corresponds to the average diameter of the primary particles.

The catalyst may be designed so that $R_l$ of Equation 6 below is within a predetermined range.

[Equation 6]

$$R_l = \frac{l + L_s}{R}$$

In Equation 6, $L_s$ is the same as $L_s$ in Equation 3 above, and a value whose unit is $\mu m$ is applied.

In addition, l in Equation 6 is a value obtained by Equation 9 below, and a value whose unit is $\mu m$ is applied.

[Equation 9]

$$l = \frac{a}{\sqrt{\pi}} - \frac{r}{2}$$

In Equation 9, a and r are the same as a and r in Equation 4, respectively.

In Equation 6, R is a value obtained by Equation 8 below, and a value whose unit is $\mu$m is applied.

[Equation 8]

$$\frac{R}{2} = \frac{\sqrt{A}}{\pi}$$

In Equation 8, A is the area A in the ratio B/A, and a value whose unit is $\mu$m$^2$ is applied.

The lower limit of $R_l$ in Equation 6 may be 0, 0.001, 0.005, 0.01, 0.05, 0.1, 0.5, 0.7, or 0.1 or so, and the upper limit thereof may be 2, 1.5, 1, 0.8, 0.6, 0.4, 0.2, 0.1, 0.08, 0.06, 0.04, or 0.02 or so. The $R_l$ may be within a range of less than or equal to, or less than any one upper limit arbitrarily selected from the above-listed upper limits while being more than or equal to, or more than any one lower limit arbitrarily selected from the above-listed lower limits. By designing the catalyst to satisfy this range, the desired catalytic activity may be obtained, and such catalytic activity may be stably maintained during the reaction process.

The lower limit of l in Equation 6 may be 0, 0.5, 1, 1.5, 2, 2.5, 3, 3.5, 4, or 4.5 or so, and the upper limit thereof may be 7.5, 7, 6.5, 6, 5.5, 5, 4.5, 4, or 3.5 or so. The l may be within a range less than or equal to, or less than any one upper limit arbitrarily selected from the above-listed upper limits; or within a range of less than or equal to, or less than any one upper limit arbitrarily selected from the above-listed upper limits while being more than or equal to, or more than any one lower limit arbitrarily selected from the above-listed lower limits. By designing the catalyst to satisfy this range, the desired catalytic activity may be obtained, and such catalytic activity may be stably maintained during the reaction process. The unit of l is nm.

The catalyst may be designed such that $R_p$ in Equation 7 below is within a predetermined range.

[Equation 7]

$$R_p = \frac{L_s}{R}$$

In Equation 7, $L_s$ and R are the same as $L_s$ and R in Equation 6, and values whose units are each $\mu$m are applied.

The lower limit of $R_p$ in Equation 7 may be 0, 0.3, 0.4, 0.5, 1, or 1.5 or so, and the upper limit thereof may be 5, 4.5, 4, 3.5, 3, 2.5, 2, 1.5, 1, or 0.5 or so. The $R_p$ may be within a range less than or equal to, or less than any one upper limit arbitrarily selected from the above-listed upper limits; or within a range of less than or equal to, or less than any one upper limit arbitrarily selected from the above-listed upper limits while being more than or equal to, or more than any one lower limit arbitrarily selected from the above-listed lower limits. By designing the catalyst to satisfy this range, the desired catalytic activity may be obtained, and such catalytic activity may be stably maintained during the reaction process.

When a virtual circle has been drawn with the center coordinate $(C_x, C_y)$ as the center and the radius as the $L_s$ in the cross-section of the catalyst particle according to one example, the range of the ratio $B_{in}/(B_{in}+B_{out})$ of the area $B_{in}$ of the region of the second metal existing inside the virtual circle and the area $B_{out}$ of the region of the second metal existing outside the circle may be adjusted.

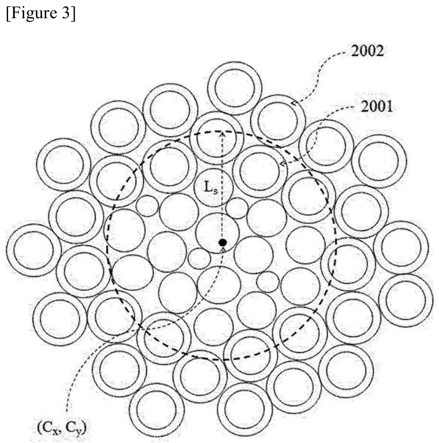

Figure 3 is an example in which a virtual circle with the center coordinates as $(C_x, C_y)$ and the radius as $L_s$ in the cross-section of the secondary particle is indicated, and according to this manner, the region of the second metal is divided into a region (2001) inside the virtual circle and a region (2002) within the virtual circle. In this state, the area of the region of the second metal in the region inside the virtual circle may be set as $B_{in}$, and the area of the region of the second metal in the region outside the virtual circle may be set as $B_{out}$. These areas $B_{in}$ and $B_{out}$ may also be obtained, for example, using the Python program, and may be obtained, for example, using a library such as scipy or numpy in the Python program. In such a program, the region of the second metal is divided into a plurality of small polygons such as triangles or quadrangles, and the $B_{in}$ and $B_{out}$ are each obtained through the areas of the respective divided polygons. The units of the areas $B_{in}$ and $B_{out}$

may each be $\mu m^2$.

The lower limit of the area ratio $B_{in}/(B_{in}+B_{out})$ obtained in the above manner may be 0, 0.05, 0.1, 0.15, 0.2, 0.25, 0.3, 0.35, or 0.4 or so, and the upper limit thereof may be 0.5, 0.45, 0.4, 0.35, 0.3, 0.25, 0.2, 0.15, 0.1, or 0.05 or so. The above ratio $B_{in}/(B_{in}+B_{out})$ may be any one number within a range of less than or equal to, or less than any one upper limit arbitrarily selected from the above-listed upper limits while being more than or equal to, or more than any one lower limit arbitrarily selected from the above-listed lower limits.

The matter that the ratio $B_{in}/(B_{in}+B_{out})$ is within the above range means that the catalyst particle is in the form of a secondary particle formed by agglomeration of multiple primary particles, and simultaneously the second metal mainly exists on the surfaces of the primary particles present on the surface of the form of the secondary particle. By controlling the form of the catalyst particles in this way, it is possible to provide a catalyst exhibiting the desired catalytic activity and stably maintaining the catalytic activity during the reaction process.

However, the foregoing is only one example of the catalyst form, and the catalyst may have various forms if the catalyst is designed according to the above-described content.

The specific type of the catalyst is not particularly limited if it satisfies the designed parameters. Particularly, the catalyst satisfying the above-described parameters is effective as $CO_2$ or CO conversion catalyst or hydrogenation catalyst particles, as described above.

Therefore, the catalyst may comprise a component included in the $CO_2$ or CO conversion catalyst or hydrogenation catalyst particles.

A representative example of such a catalyst is a Fe-based catalyst. Therefore, the first metal may be Fe. In this case, the primary particles may be Fe-based catalyst particles.

As such Fe-based catalyst particles, particles known as $CO_2$ or CO conversion catalyst or hydrogenation catalyst particles may be applied.

For example, the primary particles may be Fe bulk catalyst particles, or catalyst particles in which Fe is supported as an active component on a support. The Fe bulk catalyst particle may be a catalyst composed only of a Fe-based component as an active component without a separate support, and for example, Fe metal or a compound including Fe may be used as the bulk catalyst. In another example, the primary particles may be catalyst particles in which Fe is supported as an active component on a support. In this case, alumina or silica may be used as the support. These supports may have a large specific surface area, thereby being advantageous for supporting active components, and may be excellent in terms of catalytic activity and durability when Fe has been supported.

The primary particles may comprise an additional component in addition to the Fe. Such a component may be a component commonly known as a cocatalyst component, and may be, for example, one or more selected from the group consisting of Na, K, and Cu.

In the catalyst, it is necessary to introduce the second metal so that the above-described parameters are satisfied. Such a second metal protects the primary particles and suppresses undesirable sintering of the catalyst during the reaction process, whereby the conversion ratio of $CO_2$ and the selectivity for higher hydrocarbons (for example, hydrocarbons with 5 or more or 6 or more, or 7 or more or 8 or more carbon atoms) may be improved.

Such a second metal may comprise, for example, one or more selected from the group consisting of Al, Ce, Cu, Co, and Mo. Such a second metal may be included in the particles as a metal itself or in the form of an oxide, nitride, or acid nitride.

Such a second metal is included so that the parameters are satisfied, whereby it is possible to improve heat resistance and mechanical stability of the primary particles while improving the catalytic activity thereof without lowering.

A catalyst satisfying such parameters may be produced in the following manner.

For example, the catalyst may be produced by first producing catalyst particles (may be referred to as a precursor catalyst) (such a precursor catalyst may be in a primary particle form or a secondary particle form) including the primary particles, and introducing the second metal into such catalyst particles.

The precursor catalyst may be produced in a known manner, and may be produced by applying, for example, a precipitation method such as an incipient wetness impregnation method. In this process, by adjusting the production process, it is possible to be capable of satisfying the parameters. For example, if the size of the primary particle and/or secondary particle of the precursor catalyst is increased in the above process, the values of A and r among variables of the parameters may be increased, and the variable R may also be increased, and the $A_{avg}$ value may be controlled by controlling the number of primary particles in the catalyst. In addition, the value may also be controlled through adjustment of the size of the primary particles. The method of controlling the size of the primary particle and/or secondary particle or the number of primary particles is known.

A catalyst may be produced by introducing a second metal into such a precursor catalyst. The method of introducing the second metal is not particularly limited, and for example, the second metal may be introduced by applying a known deposition method and/or support method.

For example, among the deposition methods, a so-called ALD (Atomic Layer Deposition) method may precisely control an introduction amount of the second metal at an atomic level, and may control the introduction amount of the second metal through adjustment of the number of deposition cycles in the ALD method. In addition, by introducing the supporting method, a larger amount of the second metal may be introduced compared to deposition methods such as ALD. In this process, if the introduction amount of the second metal increases, the value of $L_s$ above tends to decrease, and even when the same method is applied, if the size of the precursor catalyst decreases, the $L_s$ value tends to decrease.

In this way, it is possible to control $L_s$, r, a, and B, and the like through control of the shape of the precursor catalyst and/or the introduction amount of the second metal.

The lower limit of the size of the catalyst particles thus produced is not particularly limited, and may be, for example, 1, 10, 30, 50, 70, or 100 or so, and the upper limit thereof may be 1,000, 800, 600, or 500 or so. The size may be within a range of less than or equal to, or less than any one upper limit arbitrarily selected from the above-listed upper limits while being more than or equal to, or more than any one lower limit arbitrarily selected from the above-listed lower limits. The size of the catalyst may be a so-called D50 particle diameter (based on volume) measured by a known laser diffraction method.

The present specification also discloses a production method of a hydrocarbon mixture applying the catalyst. Such a method may comprise a step of reacting a raw material including one or more selected from the group consisting of CO and $CO_2$ in the presence of the catalyst to obtain a hydrocarbon mixture. Such a reaction may be the so-called RWGS (Reverse water-gas shift) reaction and/or FTS (Fischer-Tropsch Synthesis) reaction.

The method for performing the reaction is not particularly limited, and a known method may be applied if the catalyst is applied.

The reaction may be performed with a high conversion ratio and selectivity. The conversion ratio and selectivity are values measured by the method described in Test Example 2 of this specification.

For example, the lower limit of the selectivity may be 40%, 45%, 50%, 52%, 54%, 56%, 58%, 60%, 62%, 64%, 66%, or 68% or so, and the upper limit thereof may be 100%, 90%, 80%, or 70% or so. The selectivity may be within a range of more than or equal to, or more than any one lower limit arbitrarily selected from the above-listed lower limits; or within a range of less than or equal to, or less than any one upper limit arbitrarily selected from the above-listed upper limits while being more than or equal to, or more than any one lower limit arbitrarily selected from the above-listed lower limits.

For example, the lower limit of the conversion ratio may be 40%, 42%, 44%, 46%, 48%, 50%, 52%, 54%, 56%, or 58% or so, and the upper limit thereof may be 100%, 90%, 80%, 70%, or 60% or so. The selectivity may be within a range of more than or equal to, or more than any one lower limit arbitrarily selected from the above-listed lower limits; or within a range of less than or equal to, or less than any one upper limit arbitrarily selected from the above-listed upper limits while being more than or equal to, or more than any one lower limit arbitrarily selected from the above-listed lower limits.

The reaction may be performed without any special limitation under conditions applicable to thermochemical conversion reactions of $CO_2$, and the like, and a known reactor may also be applied as a reactor. Conditions such as the temperature at which the reaction is performed are also not particularly limited.

**Advantageous Effects**

The present specification discloses particles. The particles can be applied to a reaction for forming hydrocarbons from a raw material including $CO_2$ and/or CO. The present specification is intended to disclose the particles, wherein the particles can obtain desired products with excellent selectivity and conversion ratios without causing undesirable agglomeration or sintering phenomena and deterioration of catalytic activity during reaction processes. For example, the particles can obtain hydrocarbons having 5 or more carbon atoms with high selectivity and conversion ratios in a reaction for obtaining hydrocarbons from a raw material including $CO_2$ and/or CO. The present specification also discloses a use of the particles.

**Description of Drawings**

Figures 1 to 4 are exemplary diagrams for explaining a shape of a catalyst.
Figures 5 and 6 are SEM images of a catalyst of an example.
Figure 7 is TEM images of a catalyst of an example.
Figure 8 is SEM images of a catalyst of a comparative example.
Figure 9 is TEM images of a catalyst of a comparative example.

**Mode for Invention**

Hereinafter, the particles, and the like are specifically described through examples, but the scope of the particles, and the like is not limited to the following examples.

**Example 1.**

A catalyst containing iron (Fe) as a first metal and aluminum (Al) as a second metal was prepared in the following manner. A solution (200 mL) in which iron nitrate hydrate ($Fe(NO_3)_3 \cdot 9H_2O$) and silica ($SiO_2$) were dissolved in distilled water was introduced into a precipitation reaction vessel. The solution was prepared by mixing the iron nitrate hydrate (A), silica (B), and distilled water (C) in a weight ratio of 222:1:200 (A: B: C). Subsequently, while the solution was stirred at about 400 rpm, the temperature was raised to about 60°C. When the temperature reached about 60°C or so, an ammonium carbonate aqueous solution (180g/L) was introduced to the precipitation reaction vessel at a rate of about 2mL/min until the pH reached about 7 or so, and further stirred at 400 rpm for 2 hours or so while maintaining the temperature at about 60°C or so to generate a precipitate. After collecting the precipitate through filtration, the precipitate was washed with distilled water and dried at 110°C for about 12 hours.

The dried precipitate was crushed using a mortar and sieved to obtain a powder. Separately, a solution was prepared by mixing distilled water (D), copper (II) nitrate trihydrate (E), and potassium nitrate (F) in a weight ratio of 4:3:50 (D: E: F). 13 g of the powder was added to 250 mL of the solution, stirred, and mixed, and then the precipitate was collected. Subsequently, the collected precipitate was dried at 110°C until the solvent was completely removed, and then calcined at 400°C for 3 hours or so to obtain secondary particles.

The obtained secondary particles were separated by a sieve to obtain particles having a particle size in a range of approximately 300μm to 500μm.

The obtained secondary particles were loaded onto a rectangular tray, and the tray was introduced into a chamber of an ALD (Atomic Layer Deposition) device. The temperature in the chamber was set to about 150°C and the process pressure was set to about 1 Torr, and one cycle was by performing processes (1) to (3) below sequentially, and the cycle was repeated 16 times (16 cycles) to introduce a second metal (aluminum) onto the surfaces of the particles.

(1) A process of pulse-injecting TMA (trimethyl aluminum) at about 20°C to the inside of the chamber together with a carrier gas (nitrogen gas) for 3 seconds (injection speed: about 100 sccm), and purging with nitrogen gas for 20 seconds after maintenance for 100 seconds, is repeated 3 times.
(2) Purging with nitrogen gas for 600 seconds after (1).
(3) A process of pulse-injecting steam instead of TMA in the process of (1) above for 3 seconds, and purging with nitrogen gas for 20 seconds after maintenance for 100 seconds, is repeated 3 times.

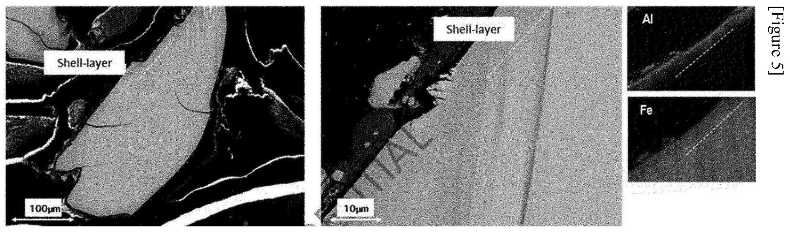

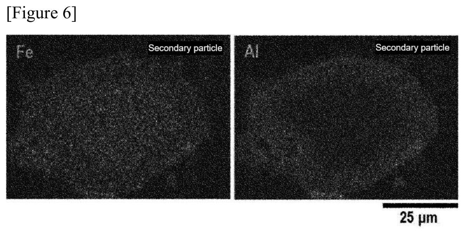

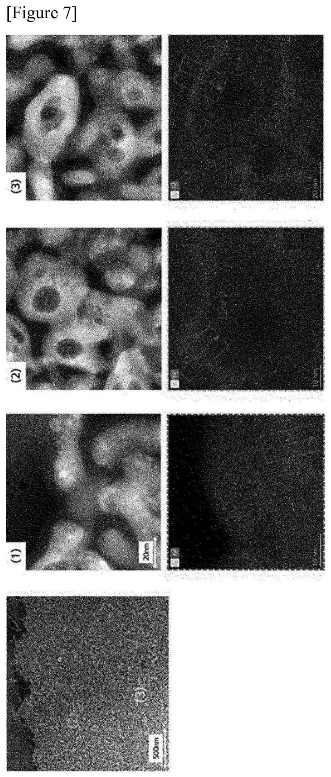

The desired catalyst particles were produced through the processes. Figures 5 and 6 are SEM (Scanning electron microscope) images obtained for the particles, and Figure 7 is TEM (Transmission Electron Microscope) images obtained for the particles.

In Figure 6, the Fe region, which is the first metal region, is identified in green, and the Al region, which is the second metal region, is identified in blue.

The catalyst particles (I) were mixed with an epoxy molding liquid and then cut to obtain a cross-section, and then the cross-sectional images were obtained through SEM (Scanning electron microscope), EDS (energy dispersive x-ray spectrometer), and EPMA (Electron Probe Micro-Analysis) analyses.

Upon photographing the SEM image, JEOL's JSM-7800F was used as the equipment, and the image was obtained by setting an acceleration voltage to 5 kV in a composition mode. To obtain clear images for the first metal (Fe) and the second metal (Al), the images were obtained by exposing the corresponding spectra for 4 hours or more using an EDS mapping analysis.

To obtain the TEM image, the catalyst was cut with FIB (Focused Ion Beam) (HELIOS 5 UX, Ga ion gun), and the TEM image was obtained for the cross-section. In the TEM image, Spectra 300 S equipment was used and the acceleration voltage condition of 200 kV was applied. The EDS mapping was performed with a Super-X EDS detector to obtain a clear image of the Fe region.

It can be confirmed from the SEM and TEM images that the obtained catalyst is in a secondary particle form in which multiple Fe-based primary particles are aggregated, and is in a form in which the second metal (Al) is introduced on the surfaces of the primary particles existing in the surface region among the multiple primary particles by the ALD.

**Example 2.**

A catalyst was obtained in the same manner as in Example 1, except that a powder having a size of 53 to 100 μm was applied as the powder for applying the ALD process by changing the sieve, and SEM and TEM images were obtained in the same manner. The obtained catalyst was in a secondary particle form in which multiple Fe-based primary particles were aggregated, and was in a form in which the second metal (Al) was introduced on the surfaces of the primary particles existing in the surface region among the multiple primary particles by the ALD.

**Example 3.**

A catalyst was obtained in the same manner as in Example 1, except that a powder having a size of 45 to 53 μm was applied as the powder for applying the ALD process by changing the sieve, and SEM and TEM images were obtained in the same manner. The obtained catalyst was in a secondary particle form in which multiple Fe-based primary particles were aggregated, and was in a form in which the second metal (Al) was introduced on the surfaces of the primary particles existing in the surface region among the multiple primary particles by the ALD.

**Example 4.**

A catalyst was obtained in the same manner as in Example 1, except that a powder having a size of 30 μm was applied as the powder for applying the ALD process by changing the sieve, and SEM and TEM images were obtained in the same manner. The obtained catalyst was in a secondary particle form in which multiple Fe-based primary particles were aggregated, and was in a form in which the second metal (Al) was introduced on the surfaces of the primary particles existing in the surface region among the multiple primary particles by the ALD.

**Example 4.**

A catalyst was obtained in the same manner as in Example 1, except that a powder having a size of 30 μm was applied as the powder for applying the ALD process by changing the sieve, and SEM and TEM images were obtained in the same manner. The obtained catalyst was in a secondary particle form in which multiple Fe-based primary particles were aggregated, and was in a form in which the second metal (Al) was introduced on the surfaces of the primary particles existing in the surface region among the multiple primary particles by the ALD.

**Example 5.**

The catalyst produced in Example 4 was further crushed to have a size of 45 μm or less, and mixed with distilled water in a weight ratio of 1:2 (catalyst: distilled water) to prepare a mixture. Thereafter, alpha alumina (mold, molded body) with a diameter of about 4.8 mm or so was mixed with the mixture, stirred at 120°C, and then calcined at 400for 3 hours after drying to produce a catalyst including the molded body. The content of the catalyst in the mixture of the catalyst and the molded body was set to about 10 wt% or so.

**Example 6.**

A catalyst was produced in the same manner as in Example 5, except that the content of the catalyst in the mixture of the catalyst and the molded body was set to about 7 to 8 wt% or so.

**Comparative Example 1.**

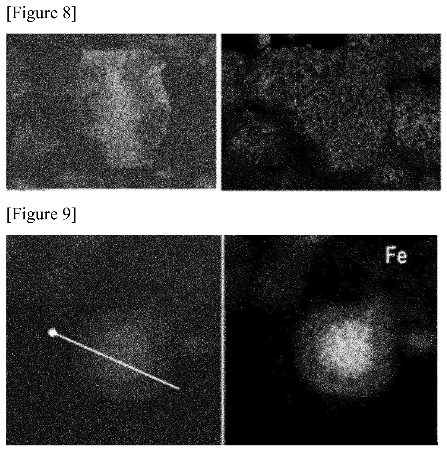

Catalyst particles were obtained in the same manner as in Example 1, except that the ALD process was not performed. Figure 8 is SEM images obtained for the particles, and Figure 9 is TEM images obtained for the particles. The method for obtaining the SEM and TEM images is the same as in Example 1. It can be confirmed from the SEM and TEM images that the obtained catalyst is in a secondary particle form in which multiple Fe-based primary particles are aggregated, but is in a form in which the second metal (Al) is not present.

**Test Example 1.**

An analysis was performed on the obtained SEM and TEM images. First, using the DBSCAN (Density-based spatial clustering of applications with noise) algorithm, the region where Fe existed (first metal region) and the region where Al existed (second metal region) in the images were defined by drawing closed curves. In this instance, when blank spaces or molded bodies containing elements other than the first metal and the second metal existed in the image, the regions where the blank spaces or molded bodies existed were excluded. For this definition, the DBSCAN was applied by setting the radius to be clustered and designating the minimum number of objects within the radius as an appropriate condition (4 to 10).

Thereafter, the image was analyzed using the scipy or numpy library of the Python program.

Using the above program, the image, which was defined as a closed curve, corresponding to the first metal region (Fe region) in the SEM image was divided into multiple small polygons such as triangles or quadrangles, and the center coordinates and areas were calculated for the respective polygons, and then the center coordinates $(C_x, C_y)$ defined by Equations 1 and 2 below were obtained (see Figure 2).

[Equation 1]

$$C_x = \frac{\sum_{i=1}^{n}(x_i \times A_i)}{\sum_{i=1}^{n} A_i}$$

[Equation 2]

$$C_y = \frac{\sum_{i=1}^{n}(y_i \times A_i)}{\sum_{i=1}^{n} A_i}$$

In Equations 1 and 2, $x_i$ and $y_i$ are the x and y coordinates of the centers of the divided individual polygons, and $A_i$ is the area of the polygon having the coordinates $x_i$ and $y_i$ as the center coordinates.

In Equations 1 and 2, n is the number of the divided polygons.

Also, the total area A of the first metal (Fe) regions was obtained by adding up the areas of the plurality of polygons. In addition, the number of the respective primary particles belonging to the first metal (Fe) region was confirmed from the TEM image, and the arithmetic average $A_{avg}$ of the areas of the first metal (Fe) regions in the respective primary particles was obtained through the areas of the first metal (Fe) regions and the particle number.

In addition, the area B of the second metal (Al) region was obtained by adding up the areas of the plurality of polygons in the same manner as the method of obtaining A above.

Thereafter, the shortest distances from the center coordinate $(C_x, C_y)$ to the region where the second metal (Al) existed were obtained at 1-degree intervals in the 360-degree direction based on the center coordinates $(C_x, C_y)$, and then $L_s$ was obtained by arithmetically averaging them.

Subsequently, the above-obtained area A was substituted into Equation 8 below to obtain R of Equation 8 below, and the area $A_{avg}$ was substituted into Equation 5 below to obtain r.

[Equation 5]

$$\frac{r}{2} = \sqrt{\frac{A_{avg}}{\pi}}$$

[Equation 8]

$$\frac{R}{2} = \frac{\sqrt{A}}{\pi}$$

In addition, using the k-Nearest Neighbors algorithm from the TEM image, the average distance a between the centers of the first metal (Fe) regions in the respective primary particles defined as closed curves in the image was also obtained. That is, the center coordinates of the first metal (Fe) region in each primary particle were obtained from the divided multiple small polygons of the first metal (Fe) region in each primary particle, and the arithmetic average of the distances between the multiple center coordinates was used as a above.

Subsequently, l of Equation 9 below was obtained by applying the average distance a and r of Equation 5 above.

[Equation 9]

$$l = \frac{a}{\sqrt{\pi}} - \frac{r}{2}$$

Table 1 below summarizes the above-obtained results.

In Table 1 below, $R_v$ is the result obtained by Equation 3 below (upon calculating, $L_s$ applies the value in the unit of $\mu$m, and A and B each apply the value in the unit of $\mu$m$^2$), $R_g$ is the result obtained by Equation 4 below (upon calculating, $L_s$, r, and a each apply the value in the unit of $\mu$m, and B applies the value in the unit of $\mu$m$^2$), $R_y$ is the result obtained by Equation 6 below (upon calculating, $L_s$, l, and R apply the values in the unit of $\mu$m), and $R_p$ is the result obtained by Equation 6 below (upon calculating, $L_s$ and R apply the values in the unit of $\mu$m).

[Equation 3]

$$R_v = \frac{-L_s + \sqrt{L_s{}^2 + \dfrac{B}{\pi}}}{2 \times \sqrt{\dfrac{A}{\pi}}}$$

[Equation 4]

$$R_g = \frac{-L_s + \sqrt{L_s{}^2 + \dfrac{B}{\pi}}}{r + a}$$

[Equation 6]

$$R_l = \frac{l + L_s}{R}$$

[Equation 7]

$$R_p = \frac{L_s}{R}$$

In Table 1, the units of A and B are each $\mu$m$^2$, the units of R and $L_s$ are each $\mu$m, and the units of a, r, and l are each nm.

[Table 1]

| | Example | | | | | | Comparative Example 1 |
|---|---|---|---|---|---|---|---|
| | 1 | 2 | 3 | 4 | 5 | 6 | |
| A | 180864 | 4776 | 1809 | 907 | 62870 | 38340 | 141791 |
| B | 24115 | 3576 | 1608 | 907 | 106785 | 162778 | 0 |
| B/A | 0.133 | 0.749 | 0.889 | 1 | 1.699 | 4.246 | 0 |
| Rv | 0.033 | 0.218 | 0.333 | 0.5 | 0.184 | 0.271 | 0 |
| Rg | 363.6 | 377.7 | 400.1 | 369.5 | 852.5 | 983.6 | 0 |

(continued)

| | | Example | | | | | | Comparative Example 1 |
|---|---|---|---|---|---|---|---|---|
| | | 1 | 2 | 3 | 4 | 5 | 6 | |
| RI | | 0.01 | 0.053 | 0.071 | 0.137 | 0.017 | 0.018 | 0.01 |
| Rp | | 0.483 | 0.321 | 0.167 | 0 | 1.064 | 1.819 | 0 |
| R | | 480 | 78 | 48 | 34 | 283 | 221 | 425 |
| Ls | | 232 | 25 | 8 | 0 | 301 | 402 | 0 |
| a | | 25 | 25 | 22 | 26 | 33 | 32 | 29 |
| r | | 19 | 20 | 18 | 20 | 28 | 29 | 24 |
| l | | 4.61 | 4.11 | 3.42 | 4.67 | 4.62 | 3.56 | 4.37 |
| a/r | | 1.32 | 1.25 | 1.22 | 1.3 | 1.18 | 1.1 | 1.21 |

**Test Example 2.**

A hydrocarbon mixture was prepared from $CO_2$ using each catalyst of Examples or Comparative Example above. 0.5 g of the catalyst was charged into a fixed-bed tubular reactor (stainless steel material, diameter 3/8 inch), and the catalyst was activated by reduction treatment for 8 hours under a CO atmosphere (>99.9 vol%) at 400°C.

Subsequently, a mixed gas in which $H_2$, $CO_2$, and $N_2$ were mixed in a volume ratio of 54:18:3 ($H_2$: $CO_2$: $N_2$) was injected into the reactor at a flow rate of 75ml/min, and the reaction was performed under conditions of 340°C and 20 bar. After the reaction, products were passed through a constant temperature water bath trap at 0°C to collect liquid hydrocarbons with 5 or more carbon atoms among the products, and gaseous products were analyzed in real time by GC (Gas Chromatography). The reaction was performed continuously for 48 hours or more.

The conversion ratio of $CO_2$ and the selectivity for hydrocarbons with 5 or more carbon atoms were confirmed by the following method.

<Conversion ratio>

$CO_2$ conversion ratio = 100 $\times$ (input $CO_2$ flow rate - exhaust $CO_2$ flow rate)/(input $CO_2$ flow rate)

<Selectivity>

$$C_{5+} \text{ selectivity} = 100\% - (\text{selectivity sum of gaseous products})$$

Selectivity of gaseous products = 100 $\times$ ($C_A H_B$ flow rate $\times$ A)/(input $CO_2$ flow rate - exhaust $CO_2$ flow rate)

The evaluation results (conversion ratio (%) and selectivity (%)) for each catalyst were summarized and described in Table 2 below.

[Table 2]

| | Example | | | | | | Comparative Example 1 |
|---|---|---|---|---|---|---|---|
| | 1 | 2 | 3 | 4 | 5 | 6 | |
| Conversion ratio | 52.5 | 50.6 | 56 | 55.9 | 55.3 | 55.6 | 44.4 |
| Selectivity | 60.1 | 67.9 | 68 | 68.1 | 68.6 | 68.8 | 46.5 |

**Claims**

1. Secondary particles in which a plurality of primary particles is aggregated, wherein the primary particles comprise a first metal,

   a second metal exists on surfaces of at least some primary particles among the primary particles,
   the first metal and the second metal are different metals, and
   $R_l$ of Equation 6 below is in a range of 0 to 2:

   [Equation 6]

   $$R_l = \frac{l + L_s}{R}$$

   wherein, $L_s$ is an average distance from the center of the first metal region of the cross-section of the secondary particle to the second metal region, R is R of Equation 8 below, and l is l of Equation 9 below:

   [Equation 8]

   $$\frac{R}{2} = \frac{\sqrt{A}}{\pi}$$

   wherein, A is an area of the first metal region in the cross-section of the secondary particle:

   [Equation 9]

   $$l = \frac{a}{\sqrt{\pi}} - \frac{r}{2}$$

   wherein, r is r of Equation 5 below, and a is an average distance between the centers of the first metal regions in the cross-section of the primary particle:

   [Equation 5]

   $$\frac{r}{2} = \sqrt{\frac{A_{avg}}{\pi}}$$

   wherein, $A_{avg}$ is an arithmetic mean of the areas of the first metal regions belonging to the respective primary particles in the cross-section of the secondary particle.

2. The particles according to claim 1, wherein $R_p$ of Equation 7 below is in a range of 0 to 5:

   [Equation 7]

   $$R_p = \frac{L_s}{R}$$

wherein, $L_s$, and R are the same as $L_s$, and R of Equation 6, respectively.

3. The particles according to claim 1, wherein in the cross-section of the secondary particle, a ratio B/A of the area B of the second metal region to the area A of the first metal region is in a range of more than 0 and 10 or less.

4. The particles according to claim 3, wherein the area A is in a range of 200 to 1,000,000 $\mu m^2$.

5. The particles according to claim 1, wherein $R_v$ of Equation 3 below is in a range of 0.01 to 1.5:

[Equation 3]

$$R_v = \frac{-L_s + \sqrt{L_s^2 + \frac{B}{\pi}}}{2 \times \sqrt{\frac{A}{\pi}}}$$

wherein, $L_s$ is an average distance from the center of the first metal region of the cross-section of the secondary particle to the second metal region, A is an area of the first metal region in the cross-section of the secondary particle, and B is an area of the second metal region in the cross-section of the secondary particle.

6. The particles according to claim 5, wherein $L_s$ is in a range of 0 to 1,000 $\mu m$.

7. The particles according to claim 1, wherein $R_g$ of Equation 4 below is in a range of 100 to 2,000:

[Equation 4]

$$R_g = \frac{-L_s + \sqrt{L_s^2 + \frac{B}{\pi}}}{r + a}$$

wherein, $L_s$ is an average distance from the center of the first metal region of the cross-section of the secondary particle to the second metal region, A is an area of the first metal region in the cross-section of the secondary particle, B is an area of the second metal region in the cross-section of the secondary particle, r is r in Equation 5, and a is an average spacing between the first metal regions in the respective primary particles in the cross-section of the secondary particle.

8. The particles according to claim 7, wherein a ratio a/r of a to r in Equation 4 is in a range of 0.1 to 2.

9. The particles according to claim 7, wherein r in Equation 4 is in a range of 1 to 500 nm.

10. The particles according to claim 1, wherein the first metal is Fe.

11. The particles according to claim 10, wherein the first metal further comprises one or more selected from the group consisting of Na, K, and Cu.

12. The particles according to claim 1, wherein the second metal is one or more selected from the group consisting of Al, Ce, Cu, Co, and Mo.

13. A method comprising a step of reacting a raw material comprising one or more selected from the group consisting of CO and $CO_2$ in the presence of the catalyst of any one of claims 1 to 12 to obtain a hydrocarbon mixture.

14. The method according to claim 13, wherein the reaction is one or more reactions selected from the group consisting of an RWGS (Reverse water-gas shift) reaction and an FTS (Fischer-Tropsch Synthesis) reaction.

15. The method according to claim 13, wherein selectivity is 40% or more.

16. The method according to claim 13, wherein a conversion ratio is 40% or more.

[Figure 1]

[Figure 2]

[Figure 3]

[Figure 4]

[Figure 5]

[Figure 6]

[Figure 7]

[Figure 8]

[Figure 9]

## INTERNATIONAL SEARCH REPORT

| International application No. |
|---|
| **PCT/KR2024/014075** |

| A. | CLASSIFICATION OF SUBJECT MATTER |
|---|---|

**B01J 35/30**(2024.01)i; **B01J 35/40**(2024.01)i; **B01J 23/745**(2006.01)i; **C07C 1/12**(2006.01)i; **C10G 2/00**(2006.01)i

According to International Patent Classification (IPC) or to both national classification and IPC

| B. | FIELDS SEARCHED |
|---|---|

Minimum documentation searched (classification system followed by classification symbols)

B01J 35/30(2024.01); B01J 19/32(2006.01); B01J 23/00(2006.01); B01J 23/755(2006.01); B01J 23/78(2006.01); B01J 29/035(2006.01); B01J 32/00(2006.01); B01J 35/02(2006.01); B01J 37/08(2006.01); C01G 35/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 촉매(catalyst), 2차 입자(secondary particle), 철(iron), 표면(surface), 탄화수소 (hydrocarbon), 피셔-트롭쉬 합성 반응(Fischer-Tropsch synthesis), 역수성 가스전환 반응(Reverse water-gas shift), 원자층 증착(atomic layer deposition)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2015-0139040 A (KOREA RESEARCH INSTITUTE OF CHEMICAL TECHNOLOGY) 11 December 2015 (2015-12-11) See paragraphs [0027]-[0033], [0037], [0047] and [0050]-[0051]; and figure 1b. | 1-10,12 |
| Y | | 11,13-16 |
| Y | JP 6466330 B2 (MITSUI MINING & SMELTING CO., LTD. et al.) 06 February 2019 (2019-02-06) See paragraphs [0015]-[0016] and [0082]; and claims 1-2. | 11,13-16 |
| A | JP 2022-090611 A (SUMITOMO METAL MINING CO., LTD.) 17 June 2022 (2022-06-17) See entire document. | 1-16 |
| A | JP 2021-186770 A (FURUKAWA ELECTRIC CO., LTD. et al.) 13 December 2021 (2021-12-13) See entire document. | 1-16 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 January 2025** | **09 January 2025** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/014075**

| C. | DOCUMENTS CONSIDERED TO BE RELEVANT | |
|---|---|---|
| Category* | Citation of document, with indication, where appropriate, of the relevant passages | Relevant to claim No. |
| A | KR 10-2017-0005724 A (UNIVERSITY OF SEOUL INDUSTRY COOPERATION FOUNDATION.) 16 January 2017 (2017-01-16)<br>See entire document. | 1-16 |

Form PCT/ISA/210 (second sheet) (July 2022)

**INTERNATIONAL SEARCH REPORT**
Information on patent family members

International application No.

**PCT/KR2024/014075**

| Patent document cited in search report | | | Publication date (day/month/year) | Patent family member(s) | | | Publication date (day/month/year) |
|---|---|---|---|---|---|---|---|
| KR | 10-2015-0139040 | A | 11 December 2015 | KR | 10-2305813 | B1 | 29 September 2021 |
| JP | 6466330 | B2 | 06 February 2019 | JP | WO2015-012189 | A1 | 02 March 2017 |
| | | | | WO | 2015-012189 | A1 | 29 January 2015 |
| JP | 2022-090611 | A | 17 June 2022 | | None | | |
| JP | 2021-186770 | A | 13 December 2021 | CN | 115697554 | A | 03 February 2023 |
| | | | | EP | 4159313 | A1 | 05 April 2023 |
| | | | | EP | 4159313 | A4 | 19 June 2024 |
| | | | | US | 2023-0211325 | A1 | 06 July 2023 |
| | | | | WO | 2021-246394 | A1 | 09 December 2021 |
| KR | 10-2017-0005724 | A | 16 January 2017 | KR | 10-1834486 | B1 | 05 March 2018 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230123492

**Non-patent literature cited in the description**

- **RA, E. C.** ; **KIM, K. Y.** ; **KIM, E. H.** ; **LEE, H.** ; **AN, K.** ; **LEE, J. S.** Recycling Carbon Dioxide through Catalytic Hydrogenation: Recent Key Developments and Perspectives.. *ACS Catal.*, 2020, vol. 10 (19), 11318-11345
- **CHOI, Y. H.** ; **JANG, Y. J.** ; **PARK, H.** ; **KIM, W. Y.** ; **LEE, Y. H.** ; **CHOI, S. H.** ; **LEE, J. S.** Carbon Dioxide Fischer-Tropsch Synthesis: A New Path to Carbon-Neutral Fuels.. *Appl. Catal., B*, 2017, vol. 202, 605-610

- **SHARMA, P.** ; **SEBASTIAN, J.** ; **GHOSH, S.** ; **CREASER, D.** ; **OLSSON, L.** Recent Advances in Hydrogenation of CO2 into Hydrocarbons via Methanol Intermediate over Heterogeneous Catalysts.. *Catal. Sci. Technol.*, 2021, vol. 11 (5), 1665-1697